(19) Europäisches Patentamt
European Patent Office
Office européen des brevets

(11) Veröffentlichungsnummer: **0 162 385 B2**

(12) # NEUE EUROPÄISCHE PATENTSCHRIFT

(45) Veröffentlichungstag der neuen Patentschrift: **14.08.91 Patentblatt 91/33**

(51) Int. Cl.$^5$: **C07C 1/207**, C07C 11/12, C07C 13/20

(21) Anmeldenummer: **85105766.1**

(22) Anmeldetag: **10.05.85**

(54) **Verfahren zur Herstellung von Dienen durch Dehydratisierung von Aldehyden.**

Die Akte enthält technische Angaben, die nach dem Eingang der Anmeldung eingereicht wurden und die nicht in dieser Patentschrift enthalten sind.

(30) Priorität: **24.05.84 DE 3419379**

(43) Veröffentlichungstag der Anmeldung: **27.11.85 Patentblatt 85/48**

(45) Bekanntmachung des Hinweises auf die Patenterteilung: **23.09.87 Patentblatt 87/39**

(45) Bekanntmachung des Hinweises auf die Entscheidung über den Einspruch: **14.08.91 Patentblatt 91/33**

(84) Benannte Vertragsstaaten: **BE CH DE FR GB IT LI NL**

(56) Entgegenhaltungen:
**DE-A- 2 163 396**
**GB-A- 2 093 060**
**SOVIET INVENTIONS ILLUSTRATED, Sektion Ch, Woche D 23, 15. Juli 1981, DERWENT PUBLICATIONS LTD., London, A 41 E 17**

(56) Entgegenhaltungen:
**PATENT ABSTRACTS OF JAPAN, unexamined applications, Field C, Band 6, Nr. 94, 2. Juni 1982, THE PATENT OFFICE JAPANESE GOVERNMENT, Seite 150 C 105**
**Chemical Abstracts 67/6:26119d**
**Chemical Abstracts 69/5: 18404u**
**Chemical Abstracts 71/6: 25042u**
**Chemical Abstracts 74/9: 41867j**
**Chemical Abstracts 85/21: 159280t**
**Chemical Abstacts 90/21: 167977u**
**Chemical Abstracts 95 (16): 139468p**
**Chemical Abstracts 88 (21): 151703z**
**Chemical Abstracts 95 (20): 176532a**
**Chemical Abstracts 95 (20): 176533b**
**Chemical Abstracts 95 (20): 176534c**
**Chemical Abstracts 95 (20): 176535d**
**Russian Chemical Reviews, 51 (12) 1982 U.D.C. 542.97**

(73) Patentinhaber: **BASF Aktiengesellschaft**
**Carl-Bosch-Strasse 38**
**W-6700 Ludwigshafen (DE)**

(72) Erfinder: **Hoelderich, Wolfgang, Dr.**
**Mannheimer Strasse 18 c**
**W-6710 Frankenthal (DE)**
Erfinder: **Merger, Franz, Dr.**
**Max-Slevogt-Strasse 25**
**W-6710 Frankenthal (DE)**
Erfinder: **Mross, Wolf Dieter, Dr.**
**Anselm-Feuerbach-Strasse 21**
**W-6710 Frankenthal (DE)**
Erfinder: **Fouquet, Gerd, Dr.**
**Maxburgstrasse 2**
**W-6730 Neustadt (DE)**

EP 0 162 385 B2

**Beschreibung**

Verfahren zur Herstellung von Dienen durch Dehydratisierung von Aldehyden

Die vorliegende Erfindung betrifft ein Verfahren zur Herstellung von Dienen durch Dehydratisierung von Aldehyden unter Verwendung von Zeolithen als Katalysatoren.

Diene sind wegen ihrer vielseitigen Verwendungsmöglichkeiten gesuchte chemische Verbindungen. Man benötigt sie z.B. in der Gummi- und Kunststoffindustrie sowie als Ausgangs- und Zwischenverbindungen bei mannigfaltigen organischen Reaktionen. Die Herstellung von Dienen aus Aldehyden durch einfache Dehydratation ist wünschenswert, da die Aldehyde z.B. über die Oxosynthese leicht zugänglich sind. Man führt sie z.B. an phosphorhaltigen Katalysatoren durch. Dabei werden als Katalysatoren z.B. Säuren, wie Phosphorsäure (DE-OS 21 63 396), Borphosphate (EP 80 449) und Ammoniumaluminiumsulfate (GB 2 063 297) verwendet. Bei diesen Verfahren ist die Wasserdampfverdünnung von Nachteil. Außerdem läßt sich eine Regenerierung der durch Koksabscheidung desaktivierten Katalysatoren nicht oder nur schwer durchführen.

Es wurde nun gefunden, daß man bei der katalytischen Dehydratisierung von Aldehyden zu Dienen bei höherer Temperatur besonders vorteilhafte Ergebnisse erzielt, wenn man die Dehydratisierung unter Verwendung von Zeolithen der aciden H-Form, die gegebenenfalls durch Alkalimetall, Erdalkalimetall, Erdmetall, Übergangsmetall, Edelmetall und/oder seltenen Erdmetallen dotiert sind, als Katalysatoren vornimmt.

Nach dem neuen Verfahren werden hohe Selektivitäten, Umsätze und Standzeiten erzielt. Ein weiterer Vorteil ist, daß hohe Selektivitäten bei langer Laufzeit des Katalysators auch unter Ausschluß von Wasserdampf erhalten werden. Ferner ist vorteilhaft, daß sich die erfindungsgemäß zu verwendenden Katalysatoren nach ihrer Desaktivierung durch Koksbildung leicht mit Luft regenerieren lassen.

Zeolithe vom NaY-Typ in H Form sind zwar schon zur Dehydratisierung von Aceton verwendet worden (SU 765 251), wobei ein Gemisch aus Allen und Methylacetylen erhalten wurde. Es konnte aber nicht erwartet werden, daß man mit Zeolithen Aldehyde so vorteilhaft und mit so vorteilhaften Selektivitäten zu Dienen dehydratisieren kann.

Der Aluminosilikatzeolith wird z. B. aus einer Aluminiumverbindung, vorzugsweise $Al(OH)_3$ oder $Al_2(SO_4)_3$, und einer Siliciumkomponente, vorzugsweise hochdispersem Siliciumdioxid in wäßriger Aminlösung, insbesondere 1,6-Hexandiamin- oder 1,3-Propandiamin- oder Triethylentetramin-Lösung mit und ohne Alkali-oder Erdalkalizusatz bei 100 bis 220°C unter autogenem Druck hergestellt. Die erhaltenen Aluminosilikatzeolithe enthalten je nach Wahl der Einsatztstoffmengen ein $SiO_2/Al_2O_3$-Verhältnis von 10 bis 40.000. Die Aluminosilikatzeolithe lassen sich auch in etherischem Medium, wie Diethylenglykoldimethylether, in alkoholischem Medium, wie Methanol bzw. 1,4-Butandiol oder in Wasser herstellen.

Der Borosilikatzeolith wird z. B. bei 90 bis 200°C unter autogenen Druck synthetisiert, indem man eine Borverbindung, z. B. $H_3BO_3$, mit einer Siliciumverbindung, vorzugsweise mit hochdispersem Siliciumdioxid in wäßriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt. Man kann bei dieser Reaktion anstelle einer wäßrigen Aminlösung eine etherische Aminlösung, z. B. mit Diethylenglykoldimethylether, oder eine alkoholische Lösung, z. B. mit 1,6-Hexandiol als Lösungsmittel verwenden.

Den Eisensilikatzeolith erhält man z. B. aus einer Eisenverbindung, vorzugsweise $Fe_2(SO_4)_3$ und einer Siliciumverbindung, vorzugsweise aus hochdispersem Siliciumdioxid in wäßriger Aminlösung, insbesondere 1,6-Hexandiamin, mit und ohne Alkali- oder Erdalkalisuzatz bis 100 bis 200°C unter autogenem Druck.

Die so hergestellten Alumino-, Boro- und Eisensilikatzeolithe können nach ihrer Isolierung, Trocknung bei 100 bis 160°C, vorzugsweise 110°C, und Calcination bei 450 bis 550°C, vorzugsweise 500°C, mit einem Bindemittel im verhältnis 90 :10 bis 40 :60 Gew.% zu Strängen oder Tabletten verformt werden. Als Bindemittel eignen sich diverse Aluminiumoxide, bevorzugt Boehmit, amorphe Alumnosilikate mit einem $SiO_2/Al_2O_3$-verhältnis von 25 :75 bis 95 :5, bevorzugt 75 :25, Silicumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Preßlinge bei 110°C/16 h getrocknet und bei 500°C/16 h calciniert. Besonders vorteilhaft lassen sich solche Katalysatoren dadurch herstellen, daß man den isolierten Alumino- bzw. Boro- bzw. Eisensilikatzeolith direkt nach der Trocknung verformt und erstmals nach der Verformung einer Calcination unterwirft. Aus den zu Strängen verformten Katalysatoren kann man durch Mahlen und Sieben Wirbelgut in der Größe von 0,1 bis 0,5 mm erhalten. Die Alumino-, Boro- und Eisensilikatzeolithe können aber auch in reiner Form, ohne Binder, als Stränge oder Tabletten eingesetzt werden.

Als Katalysatoren können auch Aluminosilikatzeolithe des Y-Typs verwendet werden, die aus Kieselsol (29 % $SiO_2$) und Natriumaluminat in wäßrigem Medium hergestellt werden. Diese Aluminosilikatzeolithe können vor ihrem Einsatz ebenfalls mit Bindern verformt werden. Die Zeolithe können auch als Mordenit-Typ vorliegen.

Liegt der Zeolith aufgrund der Art seiner Herstellung nicht in der aciden H-Form vor, sondern z. B. in der

Na-Form, dann kann diese durch Ionenaustausch mit Ammoniumionen und anschließende Calcination oder durch Behandlung mit Säuren vollkommen oder partiell in die gewünschte H-Form übergeführt werden. Man kann an den Zeolithen zur Erhöhung der Selektivität, der Standzeit und der Anzahl an Regenerierungen auch unterschiedliche Modifizierungen vornehmen. Eine geeignete Modifizierung besteht z. B. darin, daß man den unverformten oder verformten Zeolithen mit Alkalimetallen wie Na - sofern nicht schon von der Synthese her die Alkali-Form des Zeolithen vorliegt - mit Erdalkali wie Ca, Mg und Erdmetallen wie B, Tl ionenaustauschen bzw. imprägnieren kann. Insbesondere ist eine Dotierung der Zeolithe mit Übergangsmetallen, wie Mo, W, Fe, Zn, Cu, mit Edelmetallen, wie Pd und mit seltenen Erdmetallen, wie Ce, La, vorteilhaft.

Praktisch stellt man solche modifizierten Kontakte z. B. so her, daß man den verformten Pentasilzeolithen in einem Steigrohr vorlegt und bei 20 bis 100°C z. B. eine wäßrige Lösung eines Halogenids oder eines Nitrats der voranbeschriebenen Metalle darüberleitet. Ein derartiger Ionenaustausch kann z. B. an der Wasserstoff-, Ammonium- und Alkaliform des Zeolithen vorgenommen werden. Man kann die Metallaufbringung auf den Zeolithen z. B. auch so vornehmen, daß man das zeolithische Material z. B. mit einem Halogenid, einem Nitrat oder einem Oxid der voranbeschriebenen Metalle in wäßriger oder alkoholischer Lösung imprägniert. Sowohl an einen Ionenaustausch als auch an eine Imprägnierung schließt sich zumindest eine Trocknung und wahlweise eine abermalige Calcination an.

Im einzelnen verfährt man z. B. so, daß man Molybdänoxid ($MoO_3$) oder Wolframsäure ($H_2WO_4$) oder $Ce(NO_3)_3x6H_2O$ in Wasser - größtenteils zumindest - löst. Mit dieser Lösung wird dann der verstrangte oder unverstrangte Zeolith eine gewisse Zeit, ca. 30 min., getränkt. Die überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z. B. eine ammoniakalische $Pd(NO_3)_2$-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcination bei ca. 500°C kann das so gewonnene zeolithische Material mit und ohne Bindemittel zu Strängen oder Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z. B. eine ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert.

Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure- und Phosphorsäure und/oder mit Wasserdampf unterwirft. Dabei geht man vorteilhat z. B. so vor, daß man das zeolithische Pulver vor seiner Verformung mit Flußsäure (0,001 n bis 2 n, bevorzugt 0,05 n bis 0,5 n) 1 bis 3 h unter Rückfluß behandelt. Nach Abfiltrieren und Auswaschen wird bei 100 bis 160°C getrocknet und bei 400 bis 550°C calciniert. Auch eine Behandlung der Zeolithe nach ihrer Verformung mit Bindemittel mit Salzsäure kann zweckmäßig sein. Hierbei wird der Zeolith z. B. 1 bis 3 h zwischen 60 und 80° C mit einer 3 bis 25 %igen, insbesondere mit einer 12 bis 20 %igen Salzsäure behandelt, anschließend ausgewaschen, bei 100 bis 160°C getrocknet und bei 400 bis 550°C calciniert. Man kann den Zeolithen auch durch Aufbringen von Phosphorverbindungen, wie Trimethoxyphosphat, modifizieren.

Nach einer Desaktivierung der zeolithischen Katalysatoren, die beim Verfahren der Erfindung durch Koksabscheidung eintreten kann, lassen sich diese durch Abbrennen der Koksablagerung mit Luft oder mit einem Luft/$N_2$-Gemisch bei 400 bis 550° C, bevorzugt 500° C, in einfacher Weise regenerieren, wodurch sie ihre Anfangsaktivität zurückerhalten. Man kann auch durch eine partielle Verkokung (pre-coke) die Aktivität des Katalysators für ein Selektivitätsoptimum des gewünschten Reaktionsproduktes einstellen. Wird die Dehydratisierung in Gegenwart von Gasen wie Wasserstoff, Stickstoff und Wasserdampf ausgeführt, so kann damit die Produktzusammensetzung und Standzeit des Katalysators beeinflußt werden. Im allgemeinen werden die Katalysatoren wahlweise als 2 bis 4 mm Stränge, als Tabletten mit 3 bis 5 mm Durchmesser, als Pulver mit Teilchengrößen von 0,3 bis 0,5 mm oder (als Wirbelkontakt) von 0,1 bis 0,5 mm eingesetzt.

Die Dehydratisierung der Aldehyde zu den Dienen führt man an den Zeolithen vorzugsweise bei Temperaturen von 150 bis 600°C, insbesondere bei 300 bis 500°C, durch. Die Belastung (WHSV) beträgt 0,1 bis 20, vorzugsweise 0,5 bis 5 g Aldehyd pro Gramm Katalysator und Stunde. Man kann auch in der Flüssigphase, z. B. bei Temperaturen von 30 bis 300° C dehydratisieren. Das Verfahren kann chargenweise oder kontinuierlich, drucklos oder unter Druck, z. B. in einem Strömungsreaktor, Rührkessel oder Wirbelreaktor, durchgeführt werden. Unumgesetzte Aldehyde können gegebenenfalls nach der Umsetzung destillativ von den entstandenen Dienen abgetrennt und erneut für die erfindungsgemäße Umsetzung verwendet werden.

**Beispiele 1 bis 5**

Die folgenden Aldehyde wurden zu den entsprechenden Dienen dehydratisiert:

1. 2-Methylbutanal zu Isopren
2. 2-Methylpentanal zu 2-Methyl-pentadien-1,3
3. Pivalinaldehyd zu Isopren
4. Isovaleraldehyd zu Isopren
5. Cyclohexanaldehyd zu Methylcyclohexadien und/oder Methylencyclohexen.

Die Reaktionen wurden wie folgt beschrieben durchgeführt.

Der jeweilige Aldehyd wurde zum Zwecke der Dehydratisierung zum Dien unter isothermen Bedingungen in einen Rohrreaktor (Wendelform, Innendurchmesser 0,6 cm und Länge von 90 cm) eingeführt und in der Gasphase bei Temperaturen von 350 bis 450°C uber einen Zeolith-Katalysator geleitet. Die erhaltenen Reaktionsprodukte wurden destillativ aufgearbeitet und durch Siedepunkt, Brechungsindex und NMR-Spektren charakterisiert. Die quantitative Bestimmung der Reaktionsprodukte und der Ausgangsprodukte arfolgte gaschromatographisch. Die Art des Katalysators, die Temperatur, die Belastung (WHSV), der Umsatz und die Selektivität sind der folgenden Tabelle zu entnehmen. Aus Beispiel 5 ist der Einfluß der Temperatur auf den Umsatz und die Selektivität ersichtlich.

| **Beispiel** | 1 | 1 | 2 | 3 | 4 | 5 | 5 | 5 |
|---|---|---|---|---|---|---|---|---|
| Katalysator | A | C | A | A | A | A | A | A |
| Temperatur °C | 400 | 400 | 350 | 450 | 500 | 350 | 400 | 450 |
| WHSV | 2 $h^{-1}$ | 2 $h^{-1}$ | 2 $h^{-1}$ | 1,8 $h^{-1}$ | 2 $h^{-1}$ | 2 $h^{-1}$ | 2 $h^{-1}$ | 2 $h^{-1}$ |
| Umsatz % | 51,2 | 49,0 | 21,0 | 99,6 | 50,7 | 63,9 | 85,9 | 95,5 |
| Selektivität Dien % | 95,0 | 83,6 | 91,5 | 50 | 85,5 | 90,8 | 95,6 | 89,6 |

Die verwendeten Katalysatoren wurden folgendermaßen hergestellt:

**Katalysator A**

Der Katalysator wurde in einer hydrothermalen Synthese aus 64 g $SiO_2$ (hochdisperse Kieselsäure), 12,2 g $H_3BO_3$, 800 g einer wäßrigen Hexandiamin-Lösung (Mischung 50 :50 Gew.%) bei 170°C unter autogenem Druck in einem Rührautoklaven hergestellt. Nach Abfiltrieren und Auswaschen wurde das kristalline Reaktionsprodukt bei 100°C/24 h getrocknet und bei 500°C/24 h calciniert. Es wurde ein Borosilikatzeolith vom Pentasiltyp erhalten, der 94,2 Gew.% $SiO_2$ und 2,32 Gew.% $B_2O_3$ enthielt. Aus diesem Zeolithen wurden 2 mm-Stränge hergestellt, die bei 100°C getrocknet und bei 500°C/24 h calciniert wurden.

**Katalysator B**

Ein Aluminosilikatzeolith vom Pentasil-Typ wurde unter hydrothermalen Bedingungen bei autogenem Druck und 150°C aus 65 g hochdispersem $SiO_2$, 20,3 g $Al_2(SO_4)_3$ x $18H_2O$ in 1 kg einer wäßrigen 1,6-Hexandiamin-Lösung (Mischung 50 :50 Gew.%) in einem Rührautoklaven hergestellt. Nach Abfiltrieren und Auswaschen wurde das kristalline Reaktionsprodukt bei 110°C/24 h getrocknet und bei 500°C/24 h calciniert. Dieser Aluminosilikatzeolith enthielt 91,6 Gew.% $SiO_2$ und 4,6 Gew.% $Al_2O_3$.

50 g dieses Aluminosilikatzeolithen wurden mit 140 ml 0,1 n HF 1 h unter Rückfluß gekocht. Nach Abfiltrieren wurde mit Wasser neutral gewaschen, bei 110°C/16 h getrocknet und bei 500°C/5 h calciniert. Dieser Zeolith wurde mit amorphem Aluminosilikat (75 Gew.% $SiO_2$ und 25 Gew.% $Al_2O_3$) im Gewichtsverhältnis 60:40 zu 2 mm-Strängen verformt, bei 110°C/16 h getrocknet und bei 500°C/24 h calciniert.

**Katalysator C**

Es wurde wie unter Katalysator B beschrieben verfahren, wobei jedoch das 1,6-Hexandiamin durch 1,3-Propandiamin ersetzt wurde. Man erhielt einen Aluminosilikatzeolith der 90,6 Gew.% $SiO_2$ und 3,4 Gew.% $Al_2O_3$ enthielt. Aus dem Zeolith wurden durch Verformen mit Boehmit im Gewichtsverhältnis 60 :40 2 mm-Stränge hergestellt, die bei 110°C/16 h getrocknet und bei 500°C/24 h calciniert wurden. 50 g dieser Stränge wurden mit 16,5 g Trimethoxyphosphat, gelöst in 35 g Wasser, 1 h getränkt. Danach wurde der Katalysator bei 110° C/2 h getrocknet. Sein Phosphorgehalt betrug 1,5 Gew.%.

## Patentansprüche

1. Verfahren zur Herstellung von Dienen durch Dehydratisierung von Aldehyden an Katalysatoren bei höheren Temperaturen, dadurch gekennzeichnet, daß man die Dehydratisierung unter Verwendung von Zeolithen der aciden H-Form, die gegebenenfalls durch Alkalimetall, Erdalkalimetall, Erdmetall, Übergangsmetall, Edelmetall und/oder seltenen Erdmetallen dotiert sind, als Katalysatoren vornimmt.

2. Verfahren nach Anspruch 1, dadurch gekennzeichnet, daß man als Aldehyde Verbindungen der Formel

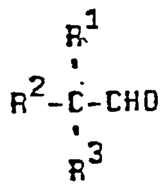

I

dehydratisiert, in der $R^1$ ein Wasserstoffatom, $R^2$ ein Wasserstoffatom, einen aliphatischen Rest mit 1 bis 10 C-Atomen und $R^3$ einen aliphatischen Rest mit 1 bis 10 C-Atomen oder einen cycloaliphatischen, aromatischen oder heterocyclischen Rest mit bis zu 10 C-Atomen bedeuten.

3. Verfahren nach Anspruch 1, dadurch gekennzeichnet, daß man als Aldehyde Verbindungen der Formel

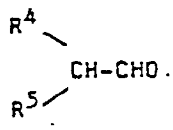

II

dehydralisiert, in der die Reste $R^4$ und $R^5$ gleich oder verschieden sind, $R^4$ einen Alkylrest mit 1 bis 3 C-Atomen oder ein Wasserstoffatom und $R^5$ einen Alkylrest mit 1 bis 8 C-Atomen oder einen Kohlenwasserstoffrest bedeutet, der zusammen mit $R^4$ einen Cyclohexylrest bildet.

4. Verfahren nach Anspruch 1, dadurch gekennzeichnet, daß man als Aldehyd 2-Methylbutanal dehydratisiert.

5. Verfahren nach Anspruch 1, dadurch gekennzeichnet, daß man als Aldehyd 2-Methylpentanal dehydratisiert.

6. Verfahren nach Anspruch 1, dadurch gekennzeichnet, daß man als Aldehyd 3-Methylbutanal dehydratisiert.

7. Verfahren nach Anspruch 1, dadurch gekennzeichnet, daß man als Aldehyd Pivalinaldehyd dehydratisiert.

8. Verfahren nach Anspruch 1, dadurch gekennzeichnet, daß man als Aldehyd Cyclohexanaldehyd dehydratisiert.

9. Verfahren nach Anspruch 1, dadurch gekennzeichnet, daß man Zeolithe vom Pentasil-Typ verwendet.

10. Verfahren nach Anspruch 1, dadurch gekennzeichnet, daß man einen Aluminosilikatzeolih verwendet.

11. Verfahren nach Anspruch 1, dadurch gekennzeichnet, daß man einen Borosilikatzeolith verwendet.

12. Verfahren nach Anspruch 1, dadurch gekennzeichnet, daß man einen Eisensilikatzeolith verwendet.

## Claims

1. A process for the preparation of a diene by dehydrating an aldehyde over a catalyst at elevated temperatures, wherein the dehydration is carried out using a zeolite of the acidic H form which may be doped with alkali metal, alkaline earth metal, earth metal, transition metal, precious metal or rare earth metals, as catalyst.

2. A process as claimed in claim 1, wherein the aldehyde dehydrated is a compound of the formula

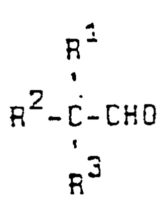

I

where $R^1$ is hydrogen, $R^2$ is hydrogen or an aliphatic radical of 1 to 10 carbon atoms and $R^3$ is an aliphatic radical of 1 to 10 carbon atoms or a cycloaliphatic, aromatic or heterocyclic radical of up to 10 carbon atoms.

3. A process as claimed in claim 1, wherein the aldehyde dehydrated is a compound of the formula

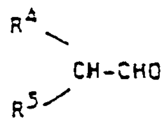 II

where $R^4$ and $R^5$ are identical or different, $R^4$ is alkyl of 1 to 3 carbon atoms or hydrogen, $R^5$ is alkyl of 1 to 8 carbon atoms or a hydrocarbon radical which together with $R^4$ forms a cyclohexyl radical.

4. A process as claimed in claim 1, wherein the aldehyde dehydrated is 2-methylbutanal.

5. A process as claimed in claim 1, wherein the aldehyde dehydrated is 2-methylpentanal.

6. A process as claimed in claim 1, wherein the aldehyde dehydrated is 3-methylbutanal.

7. A process as claimed in claim 1, wherein the aldehyde dehydrated is pivalaldehyde.

8. A process as claimed in claim 1, wherein the aldehyde dehydrated is cyclohexanecarbaldehyde.

9. A process as claimed in claim 1, wherein a zeolite of the pentasil type is used.

10. A process as claimed in claim 1, wherein an aluminosilicate zeolite is used.

11. A process as claimed in claim 1, wherein a borosilicate zeolite is used.

12. A process as claimed in claim 1, wherein an iron silicate zeolite is used.

## Revendications

1. Procédé de préparation de diènes par déshydratation d'aldéhydes sur des catalyseurs à températures élevées, caractérisé en ce que l'on effectue la déshydratation en utilisant comme catalyseurs des zéolithes sous forme H acide qui sont éventuellement dopées par des métaux alcalins, des métaux alcalinoterreux, des métaux terreux, des métaux de transition, des métaux précieux et/ou des terres rares.

2. Procédé suivant la revendication 1, caractérisé en ce que l'on déshydrate, comme aldéhydes, des composés de formule

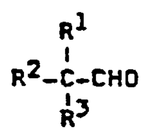 I,

dans laquelle $R^1$ est un atome d'hydrogène, $R^2$ est un atome d'hydrogène, un radical aliphatique contenant 1 à 10 atomes de carbone et $R^3$ est un radical aliphatique contenant 1 à 10 atomes de carbone, ou un radical cycloaliphatique, aromatique ou hétérocyclique contenant jusqu'à 10 atomes de carbone.

3. Procédé suivant la revendication 1, caractérisé en ce que l'on déshydrate, comme aldéhydes, des composés de formule

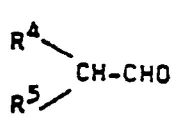 II.

dans laquelle les radicaux $R^4$ et $R^5$ sont identiques ou différents, $R^4$ représente un radical alkyle contenant 1 à 3 atomes de carbone, ou un atome d'hydrogène, et $R^5$ représente un radical alkyle contenant 1 à 8 atomes de carbone ou un radical d'hydrocarbure qui, conjointement avec $R^4$, forme un radical cyclohexyle.

4. Procédé suivant la revendication 1, caractérisé en ce que l'on déshydrate, comme aldéhyde, le 2-méthylbutanal.

5. Procédé suivant la revendication 1, caractérisé en ce que l'on déshydrate, comme aldéhyde, le 2-méthylpentanal.

6. Procédé suivant la revendication 1, caracterisé en ce que l'on déshydrate, comme aldéhyde, le 3-méthylbutanal.

7. Procédé suivant la revendication 1, caractérisé en ce que l'on déshydrate, comme aldéhyde, l'aldéhyde pivalique.

8. Procédé suivant la revendication 1, caractérisé en ce que l'on déshydrate, comme aldéhyde, le cyclohexane-aldéhyde.

9. Procédé suivant la revendication 1, caractérisé en ce que l'on utilise des zéolithes du type Pentasil.

10. Procédé suivant la revendication 1, caractérisé en ce que l'on utilise, comme zéolithe un aluminosilicate.

11. Procédé suivant la revendication 1, caractérisé en ce que l'on utilise, comme zéolithe, un borosilicate.

12. Procédé suivant la revendication 1, caractérisé en ce que l'on utilise, comme zéolithe, un silicate de fer.